# EUROPÄISCHE PATENTSCHRIFT

(11) **EP 3 085 343 B1**
(45) Veröffentlichungstag und Bekanntmachung des Hinweises auf die Patenterteilung: **20.11.2019**
(21) Anmeldenummer: 16166186.3
(22) Anmeldetag: 20.04.2016
(51) Int. Cl.: A61F 5/01

(54) **ORTHESE ZUR BEÜBUNG EINES SPRUNGGELENKS**
ORTHOSIS FOR EXERCISING AN ANKLE JOINT
ORTHESE DE REEDUCATION DE CHEVILLE

(30) Priorität: 24.04.2015 DE 102015207584
(43) Veröffentlichungstag der Anmeldung: 26.10.2016
(73) Patentinhaber: Pohlig GmbH, 83278 Traunstein (DE)
(72) Erfinder: Kienzle, Christian, 83064 Raubling (DE); Pohlig-Wetzelsperger, Claudia, 83278 Traunstein (DE)
(74) Vertreter: Pfenning, Meinig & Partner mbB

(56) Entgegenhaltungen:
- WO-A1-91/13604
- WO-A1-95/34257
- US-A- 3 086 521
- US-A1- 2008 082 034
- US-A1- 2013 226 059

## Beschreibung

Die vorliegende Erfindung bezieht sich auf eine Orthese zur Beübung eines Sprunggelenks zur, während oder nach einer Korrektur einer Fehlstellung des Sprunggelenks, beispielsweise bei einer dynamischen Klumpfuß- oder Spitzknickfußfehlstellung.

Im Stand der Technik sind Orthesen bekannt, mit denen eine Fehlstellung des Sprunggelenks gezielt und einfach behandelt werden kann. Diese weisen meist ein oberes Schalenteil zur Aufnahme eines Unterschenkels und ein unteres Schalenteil zur Aufnahme eines Fußes auf. Die beiden Schalenteile sind über ein Orthesengelenk zueinander verdrehbar und verschiebbar verbunden, um eine Korrekturstellung für das Sprunggelenk genau einzustellen. Das Orthesengelenk ist ferner mit einer Feststelleinrichtung ausgestattet, um die eingestellte Korrekturstellung des Sprunggelenks beizubehalten.

Problematisch hierbei ist jedoch, dass das Sprunggelenk für eine längere Zeit ruhig gestellt wird, um die am Sprunggelenk beteiligten Bänder und Muskeln an die korrigierte Gelenkstellung zu gewöhnen. Durch die länger anhaltende Ruhigstellung kann es zu einer Versteifung des behandelten Gelenks kommen. Diese Versteifung kann dann nach der Korrektur der Gelenkfehlstellung gegebenenfalls durch eine krankengymnastische Behandlung rückgängig gemacht werden, was jedoch eine Verlängerung der Therapiedauer bedeutet. Ferner kann die Versteifung auch mit Gelenkschmerzen verbunden sein.

Weiterhin sind bereits Orthesen bekannt, welche eine Beübung des oberen Teils des Sprunggelenks (auch oberes Sprunggelenk genannt) während oder nach der Korrektur einer Fehlstellung des Sprunggelenks ermöglichen. Hierzu sind die Orthesen mit Orthesengelenken ausgestattet, die eine Bewegung des oberen Sprunggelenks entlang vorbestimmter Freiheitsgrade und in einem bestimmten Bewegungsbereich erlauben.

Das obere Sprunggelenk wird von den distalen (unteren) Enden des Wadenbeins und des Schienbeins sowie das Sprungbein gebildet. Das untere Sprunggelenk wird u.a. vom Sprungbein und dem unterhalb (distal) des Sprungbeins gelegenen Fersenbeins gebildet.

Orthesen zur Beübung des oberen Sprunggelenks können jedoch nicht auch zur Beübung des unteren Sprunggelenks verwendet werden, da das obere und das untere Sprunggelenk verschieden verlaufende Drehachsen aufweisen. So verläuft etwa die Drehachse des oberen Sprunggelenks meist in einem Winkel von 68° bis 88° zur Längsachse des Unterschenkelknochens durch den Knöchel. Die Drehachse des unteren Sprunggelenks verläuft dagegen schräg in einem nach vorne und nach oben offenen Winkel von etwa 42° und in einem ebenfalls nach vorne und zur Großzehe hin offenen Winkel von etwa 16° zur Längsachse des Fußes. Daher ist eine völlig anders konstruierte Orthese zur Beübung des unteren Sprunggelenks als zur Beübung des oberen Sprunggelenks notwendig.

Dokument US 2013/0226059 A1 offenbart eine Knöchel-Fuß-Orthese, die einen ersten Teil zur Verbindung mit einem Beingurt, einen zweiten Teil zur Verbindung mit einem Fußgurt und einem Verbindungsteil zum Verbinden des ersten und zweiten Teils. Der Verbindungsteil weist einen ersten Einsteller auf, der in einer Einstellbedingung eine erste relative Anpassungsbewegung des ersten Teils und des zweiten Teils um eine erste Vorrichtungsachse erlaubt. Der Verbindungsteil weist einen zweiten Einsteller auf, der in einer Einstellbedingung eine zweite relative Anpassungsbewegung des ersten Teils und des zweiten Teils um eine zweite Vorrichtungsachse ermöglicht. Bei Verwendung an einem Patienten entspricht die erste Vorrichtungsachse im Wesentlichen einer dominierenden anatomischen Achse des Subtalar-Gelenks und die zweite Vorrichtungsachse im Wesentlichen einer dominierenden anatomischen Achse des Tibiotalar-Gelenks.

Dokument WO 91/13604 offenbart eine dynamische Schiene, die eine Gelenk- und insbesondere eine Körperteilverbindung stützt und die in der Lage ist, das Gelenk zyklisch durch einen programmierbaren Bewegungsbereich zu bewegen, während eine normale Gelenkkomponentenbewegung und eine Triplanarbewegung auftritt.

Dokument US 2008/0082034 A1 bezieht sich auf ein Knöchel-Derotations- und Subtalar-Stabilisierungssystem, das einen schwenkbaren, verstellbaren, schrägen Bindegurt aufweist, der sowohl an einem Vorderfußteil als auch an einer Beinkomponente verankert ist und der an einen schwenkbaren, spannbaren Derotationsgurt angrenzt, welcher hinter dem Beim entlang läuft und an der Beinkomponente befestigt ist. Das System begrenzt eine entgegengesetzt gerichtete Drehung zwischen dem Fuß und dem Bein.

Dokument WO 95/34257 offenbart eine Haltekonstruktion, die sowohl als Verhinderungseinrichtung als auch als Heilmittel geeignet ist. Die Haltekonstruktion umfasst jeweils drei Teile, die jeweils miteinander gelenkig verbunden sind. Die drei Teile sind derart angeordnet, um die Verschiebung des ersten Teils in der Sagittalebene des Tibio-Tarsal-Gelenks und andererseits am oberen Ende der Pedalschale an der Stelle des V vorzusehen, so daß die Pedalschale Evers- und Inversionsbewegungen in Bezug auf die durch die Stange und die Sub-Astragalische Schale gebildete Anordnung bewirken können.

Ausgehend vom Stand der Technik ist es daher die Aufgabe der vorliegenden Erfindung, eine Orthese bereitzustellen, die eine Beübung oder auch die Führung des unteren Sprunggelenks zur, während oder nach einer Korrektur einer Fehlstellung des Sprunggelenks ermöglicht und somit eine Versteifung des unteren Sprunggelenks verhindert.

Diese Aufgabe wird durch eine gemäß Anspruch 1 ausgestaltete Orthese gelöst. Vorteilhafte Weiterbildungen der erfindungsgemäßen Orthese können den abhängigen Ansprüche 2 bis 10 entnommen werden.

Erfindungsgemäß weist die Orthese zur Beübung eines ein oberes und ein unteres

Sprunggelenk aufweisenden menschlichen Sprunggelenks in einer korrigierten Stellung einen Fußteil zur Aufnahme eines Fußes, wobei der Fußteil eine Fußteiloberseite und einen unteren Fersenbereich aufweist, und einen Unterschenkelteil zur Aufnahme eines Unterschenkels auf. Der Fußteil und der Unterschenkelteil sind derart zueinander ausgerichtet, dass das Sprunggelenk in eine korrigierte Stellung bringbar ist. Hierbei kann sowohl das obere als auch das untere Sprunggelenk in eine korrigierte Stellung gebracht werden. Die erfindungsgemäße Orthese weist weiterhin mindestens ein erstes Orthesengelenk auf, welches eine Drehung des Fußteils relativ zum Unterschenkelteil um eine erste Drehachse ermöglicht. Die erste Drehachse schneidet den Fußteil an der Fußteiloberseite und in dem unteren Fersenbereich. Weiterhin entspricht die erste Drehachse dabei einer Drehachse des unteren Sprunggelenks in der korrigierten Stellung des Sprunggelenks. Die erfindungsgemäße Orthese zeichnet sich dadurch aus, dass das erste Orthesengelenk einen Ringbügel umfasst, an dessen Enden der Fußteil um die erste Drehachse drehbar befestigt ist.

Damit ist es mit der erfindungsgemäßen Orthese möglich eine Fehlstellung des Sprunggelenks, insbesondere des oberen und/oder unteren Sprunggelenks, zu korrigieren und gleichzeitig eine Beweglichkeit des unteren Sprunggelenks um die erste Drehachse zu gewährleisten. Somit kann das untere Sprunggelenk in der korrigierten Stellung beübt und eine Versteifung des Sprunggelenks verhindert werden. Dies verkürzt die Behandlungsdauer und verbessert das Wohlbefinden des Patienten.

In einer besonders vorteilhaften Ausgestaltung der Erfindung kann die erste Drehachse in einem Winkel zwischen 30° und 50°, vorzugsweise zwischen 39° und 42°, in Richtung einer Fußteiloberseite zu einer Horizontalebene durch den Fußteil und in einem Winkel zwischen 10° und 20°, vorzugsweise zwischen 12° und 16°, in Richtung einer Fußteilinnenseite zu einer Sagittalebene durch den Fußteil durch einen Fersenbereich des Fußteils verlaufen, in dem das untere Sprunggelenk positionierbar ist.

Die Sagittalebene durch den Fußteil bezeichnet dabei eine Ebene, die eine Längsachse des Fußteils sowie eine Längsachse des Unterschenkelteils enthält, wenn sich das Fußteil in einer gegenüber dem Unterschenkelteil nicht verdrehten Position befindet. Die Sagittalebene zerschneidet den Fußteil und den Unterschenkelteil demnach der Länge nach in zwei Hälften. Die Fußteilinnenseite entspricht einer zur Körpermitte hin ausrichtbaren Seite des Fußteils, wenn die Orthese an einen zu behandelnden Fuß angelegt wird. Die Horizontalebene durch den Fußteil bezeichnet eine Ebene, welche die Längsachse des Fußteils enthält und weitgehend senkrecht zur Längsachse des Unterschenkelteils verläuft. Die Fußteiloberseite entspricht einer zum Fußspann hin ausrichtbaren Seite des Fußteils, wenn die Orthese an einen zu behandelnden Fuß angelegt wird. Die erste Drehachse verläuft daher schräg von oberhalb einem Großzehenbereich zu einem unteren, äußeren Fersenbereich durch das untere Sprunggelenk.

Durch diese Ausrichtung der ersten Drehachse des ersten Orthesengelenks, so dass diese mit der Drehachse des unteren Sprunggelenks in einer korrigierten Stellung weitgehend übereinstimmt, kann eine Beweglichkeit des unteren Sprunggelenks um seine Drehachse in einer korrigierten Stellung ermöglicht werden, und das Sprunggelenk in der korrigierten Stellung beübt werden.

Das erste Orthesengelenk kann insbesondere derart ausgestaltet sein, dass sich eine Position der ersten Drehachse auch nachträglich verschieben und an ein zu behandelndes Sprunggelenk genauer anpassen lässt.

In einer weiteren vorteilhaften Ausgestaltung der Erfindung kann die Orthese ein zweites Orthesengelenk aufweisen, welches eine Drehung des Fußteils relativ zum Unterschenkelteil um eine zweite Drehachse ermöglicht, wobei die zweite Drehachse einer Drehachse des oberen Sprunggelenks in der korrigierten Stellung des Sprunggelenks entspricht. Hierdurch wird zusätzlich zur Beweglichkeit des unteren Sprunggelenks auch eine Beweglichkeit des oberen Sprunggelenks während oder nach einer Korrektur einer Fehlstellung des oberen und/oder unteren Sprunggelenks gewährleistet, und zusätzlich eine Beübbarkeit des oberen Sprunggelenks ermöglicht.

Dabei kann die zweite Drehachse weitgehend senkrecht, vorzugsweise in einem Winkel zwischen 70° und 90°, insbesondere in einem Winkel von 80° zur Längsachse des Unterschenkelteils durch einen oberhalb des Fersenbereichs des Fußteils gelegenen Knöchelbereich der Orthese, in dem das obere Sprunggelenk positionierbar ist, verlaufen.

Das zweite Orthesengelenk kann insbesondere weiter derart ausgestaltet sein, dass sich eine Position der zweiten Drehachse auch nachträglich verschieben und an ein zu behandelndes Sprunggelenk genauer anpassen lässt.

In einer bevorzugten Ausgestaltung der Erfindung sind die Enden des Ringbügels vorzugsweise in Haltepunkten des Fußteils befestigt, in denen die erste Drehachse das Fußteil schneidet. In diesem Fall liegt ein oberer Haltepunkt auf der Fußteiloberseite und ein unterer Haltepunkt an einer der Körpermitte abgewandten Fußteilaußenseite des Fersenbereichs. Der Ringbügel kann dann entlang der Fußteilinnenseite vom ersten zum zweiten Haltepunkt verlaufen.

Der Ringbügel kann starr mit dem Unterschenkelteil verbunden sein oder über das zweite Orthesengelenk mit dem Unterschenkelteil verbunden sein, je nachdem, ob ein zweites Orthesengelenk vorhanden ist oder nicht.

Insbesondere kann der Unterschenkelteil über eine Längsschiene mit dem Ringbügel verbunden sein, wobei das zweite Orthesengelenk dann in einem Verbindungspunkt zwischen der Längsschiene und dem Ringbügel angeordnet ist. Die Längsschiene und der Verbindungspunkt sind ebenso wie der Ringbügel vorzugsweise an einer der Körpermitte zugewandten Innenseite der Orthese angeordnet. Dies trägt zur Verbesserung der optischen Erscheinung des Patienten bei, wenn er die Orthese über einen längeren Zeitraum trägt und steigert somit den Komfort des Patienten.

Alternativ wäre es auch denkbar die Längsschiene, den Verbindungspunkt und den Ringbügel an einer der Körpermitte abgewandten Außenseite anzuordnen. Dies würde die eine mögliche Verletzungsgefahr des anderen Beins durch einen Kontakt mit dem Verbindungspunkt, der aufgrund der Konstruktion der Orthese etwas zur Außenseite vom Fußteil herausragen würde, verringern.

In einer weiteren vorteilhaften Ausgestaltung der Orthese kann das erste und/oder das zweite Orthesengelenk Anschlagelemente zur Einschränkung einer Bewegung des Fuß- und des Unterschenkelteils zueinander um die erste und/oder die zweite Drehachse aufweisen. Auf diese Weise kann der Bewegungsbereich des Fuß- und/oder Unterschenkelteils auf einen bestimmten Winkelbereich eingeschränkt werden, um einerseits eine effiziente und exakte Korrektur der Fehlstellung des unteren bzw. oberen Sprunggelenks zu erreichen und andererseits trotzdem eine gewisse Beweglichkeit zu gewährleisten.

Vorzugsweise weist das erste Orthesengelenk und/oder das zweite Orthesengelenk ein Kugelgelenk oder ein Scharniergelenk auf. Ein Kugelgelenk hat den Vorteil, dass auch bei kleinen Fertigungstoleranzen eine leichtgängige Beweglichkeit des Fußteils gegenüber dem Unterschenkelteil ermöglicht wird, da ein Kugelgelenk eine in einem gewissen Winkelbereich variable Drehachse aufweist. Dagegen kann mit einem Scharniergelenk die Drehachse exakt eingestellt und somit eine Beweglichkeit um eine exakt eingestellte Drehachse gewährleistet werden.

Weiterhin ist es möglich das erste Orthesengelenk und/oder das zweite Orthesengelenk mit einem Motor zu einer elektronisch gesteuerten Bewegung des Fuß- und Unterschenkelteils zueinander um die erste und/oder die zweite Drehachse zu koppeln. Hierdurch kann das Sprunggelenk automatisch beübt werden, ohne das der Patient oder ein Orthopäde das Fußteil gegenüber dem Unterschenkelteil selbst per Hand bewegen muss.

Das erste und/oder das zweite Orthesengelenk können auch feststellbar ausgestaltet sein, falls eine Ruhigstellung des unteren und/oder des oberen Sprunggelenks über einen gewissen Zeitraum erwünscht ist, damit sich beispielsweise Bänder und Muskeln an eine Korrekturstellung gewöhnen können.

Offenbart wird ebenfalls ein Verfahren zur Beübung eines ein oberes und ein unteres Sprunggelenk aufweisenden menschlichen Sprunggelenks in einer korrigierten Stellung mit einer vorstehend beschriebenen Orthese, wobei der Fußteil an den Fuß und der Unterschenkelteil an den Unterschenkel angebracht wird, hierdurch das Sprunggelenk in die korrigierte Stellung gebracht wird und der Fußteil relativ zum Unterschenkelteil um die erste und/oder zweite Drehachse hin und her gedreht wird.

Ferner wird offenbart eine Verwendung einer vorstehend beschriebenen Orthese zur Korrektur einer Fehlstellung eines menschlichen Sprunggelenks, insbesondere zur Korrektur eines Klumpfußes und/oder eines Knickfußes

Weitere mögliche Ausgestaltungsmerkmale der erfindungsgemäßen Orthese und des erfindungsgemäßen Verfahrens können den vorveröffentlichten deutschen Patentanmeldungen DE 10 2012 002 552 A1 und DE 10 2012 002 554 A1 entnommen werden, welche hiermit als umfasst angesehen werden sollen.

Im Folgenden werden einige Beispiele einer erfindungsgemäßen Orthese anhand von Figuren detaillierter beschrieben. Dabei werden verschiedene erfindungswesentliche oder vorteilhafte Elemente im Rahmen dieser Beispiele genannt, wobei auch einzelne dieser Elemente als solche zur Weiterbildung der Erfindung - auch herausgelöst aus dem Kontext der Beispiele - verwendet werden können. Ferner entsprechen in den Figuren gezeigte gleiche oder ähnliche Elemente gleichen oder ähnlichen Bezugszeichen.

Es zeigen
- Figur 1: ein erstes Ausführungsbeispiel einer erfindungsgemäßen Orthese in einer Seitenansicht mit Blick auf eine proximale Seite (Innenseite) der Orthese,
- Figur 2: ein zweites Ausführungsbeispiel einer erfindungsgemäßen Orthese in einer Seitenansicht mit Blick auf eine Innenseite der Orthese,
- Figur 3: die Orthese aus Figur 1 oder Figur 2 in einer Hinteransicht mit Blick auf einen Fersenbereich der Orthese,
- Figur 4: die Orthese aus Figur 1 oder Figur 2 in einer Seitenansicht mit Blick auf eine distale Seite (Außenseite) der Orthese
- Figur 5: die Orthese aus Figur 1 oder Figur 2 in einer Vorderansicht mit Blick auf einen Zehenbereich der Orthese,
- Figur 6: die Orthese aus Figur 1 oder Figur 2 in einer Draufsicht mit Blick auf den Fußteil,
- Figur 7: ein erfindungsgemäßes erstes und zweites Orthesengelenk gemäß dem ersten Ausführungsbeispiel in einer Perspektivansicht und
- Figur 8: ein erfindungsgemäßes erstes und zweites Orthesengelenk gemäß dem zweiten Ausführungsbeispiel in einer Perspektivansicht.

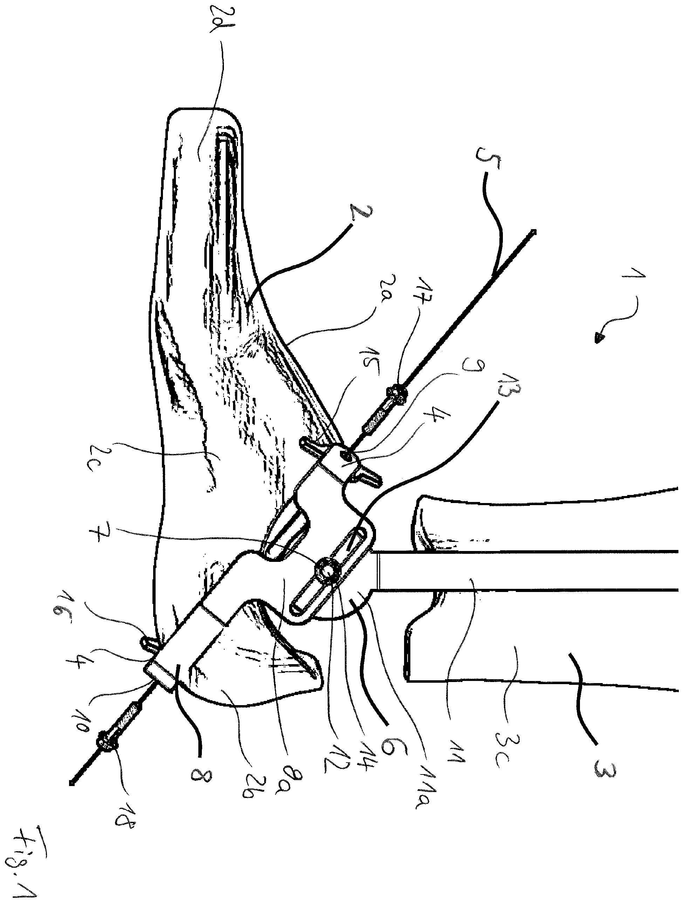

Figur 1 zeigt ein erstes Ausführungsbeispiel einer erfindungsgemäßen Orthese 1 in einer Seitenansicht mit Blick auf eine einer Körpermitte zugewandten Innenseite der Orthese 1. Die Orthese 1 weist einen Fußteil 2 und einen mit dem Fußteil 2 beweglich verbundenen Unterschenkelteil 3 auf. Der Fußteil 2 ist als geschlossene Ringfassung ausgebildet und weist im Bereich des Sprunggelenks eine Öffnung zum Anlegen des Fußteils 2 an einen Fuß auf. Der Unterschenkelteil 3 weist eine weitgehend zylindrische Form auf, die sowohl auf einer dem Fußteil 2 zugewandten Seite als auch auf einer dem Fußteil 2 abgewandten Seite jeweils eine große Öffnung zum Anlegen des Unterschenkelteils 3 an einen Unterschenkel aufweist.

Im Bereich des zu positionierenden Sprunggelenks weist der Fußteil 2 ein erstes Orthesengelenk 4 in Form eines um eine erste Drehachse 5 drehbar gelagerten Ringbügels 8 auf. Der Ringbügel 8 verläuft an einer der Körpermitte zugewandten, proximalen Innenseite 2c des Fußteils 2. Der Fußteil 2 ist somit an dem Ringbügel 8 um die erste Drehachse 5 drehbar. Die erste Drehachse 5 verläuft dabei schräg durch den Fußteil 2, von in etwa oberhalb eines der Körpermitte zugewandten, proximalen (innenliegenden) Großzehenbereichs 2d des Fußteils 2 zu einem unteren, distalen (äußeren) Fersenbereich 2b. Mit anderen Worten, zöge man eine längsseitige Mittellinie durch den Bereich des Fußteils 2, in dem das untere Sprunggelenk positioniert werden soll, dann verläuft die erste Drehachse 5 in einem Winkel von etwa 42° in Richtung einer der Körpermitte zugewandten, proximalen Fußteiloberseite 2a zu der Mittellinie und in einem Winkel von etwa 16° zur Mittellinie in Richtung einer der Fußteilaußenseite 2e abgewandten Fußteilinnenseite 2c. Diese erste Drehachse 5 entspricht in etwa der Drehachse des in der Orthese 1 zu positionierenden unteren Sprunggelenks. Der Ringbügel 8 ist an seinem oberen Ende an einem oberen Haltepunkt 9 mittels einer Schraube 17 und an seinem unteren Ende an einem unteren Haltepunkt 10 mittels einer Schraube 18 drehbar befestigt. Der obere Haltepunkt 9 befindet sich an einer Stelle des Fußteils 2, in der die erste Drehachse 5 den Fußteil 2 an der Fußteiloberseite 2a schneidet. Der untere Haltepunkt 10 befindet sich an einer Stelle, in der die erste Drehachse 5 den Fußteil 2 in dem unteren Fersenbereich 2b schneidet. Der Ringbügel 8 bildet somit ein Scharniergelenk, welches um die erste Drehachse 5 drehbar ist. Zwischen den Enden des Ringbügels 8 und dem Fußteil 2 sind jeweils einstellbare Anschlagelemente 15, 16 zur Einschränkung eines Winkelbereichs der Drehbewegung um die erste Achse 5 angeordnet.

Zwischen den Enden des Ringbügels 8 weist der Ringbügel 8 eine auf einer Fußteilinnenseite 2c gelegene Ausbuchtung 8a in Richtung des Unterschenkelteils 3 zur Verbindung des Ringbügels 8 mit dem Unterschenkelteil 3 auf. Dazu ist an einer der Körpermitte zugewandten, proximalen Innenseite des Unterschenkelteils 3 parallel zu einer Längsachse des Unterschenkelteils 3 eine Längsschiene 11 am Unterschenkelteil 3 starr befestigt. Die Längsschiene 11 überlappt im Bereich der Ausbuchtung 8a mit dem Ringbügel 8. Im Bereich der Überlappung weist die Längsschiene 11 ein kreisscheibenförmiges Ende 11a auf, das mit der Ausbuchtung 8a des Ringbügels 8 in einem Verbindungspunkt 12 über eine Schraube 14 zueinander um eine zweite Drehachse 7 drehbar verbunden ist. Die Ausbuchtung 8a und das kreisscheibenförmige Ende 11a bilden ein zweites Orthesengelenk 6, das um die zweite Drehachse 7 drehbar ist und als Scharniergelenk ausgestaltet ist. Die zweite Drehachse 7 verläuft in etwa senkrecht zu den Längsachsen des Fußteils 2 und des Unterschenkelteils 3 durch einen Bereich der Orthese 1, in dem das obere Sprunggelenk positionierbar ist.

Der Verbindungspunkt 12, welcher gleichzeitig die Position der Achse des zweiten Orthesengelenks 6 darstellt, befindet sich in einer länglichen Nut 13, die in der Ausbuchtung 8a des Ringbügels 8 angeordnet ist. Die Nut 13 verläuft in etwa parallel zum Ringbügel 8 und ermöglicht eine nachträgliche Einstellung der Position der zweiten Drehachse 7, um die Orthese 1 genauer an ein zu behandelndes Sprunggelenk anpassen zu können.

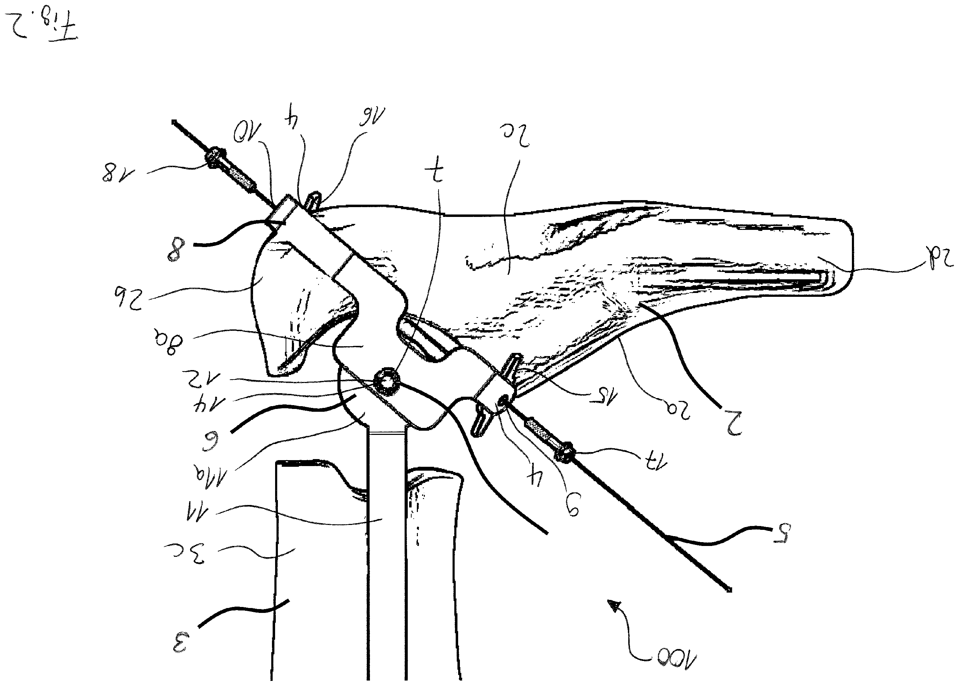

Figur 2 zeigt ein zweites Ausführungsbeispiel einer erfindungsgemäßen Orthese 100 in einer Seitenansicht mit Blick auf eine der Körpermitte zugewandte Innenseite der Orthese 100. Die Orthese 100 weist ebenso wie die Orthese 1 in Figur 1 einen Fußteil 2, einen mit dem Fußteil 2 beweglich verbundenen Unterschenkelteil 3 sowie ein erstes Orthesengelenk 4 und eine zweites Orthesengelenk 6 auf. Die Orthese 100 in Figur 2 unterscheidet sich lediglich dadurch von der Orthese 1 der Figur 1, dass die Ausbuchtung 8a des Ringbügels 8 keine Nut zur Verstellung der Position der Achse des zweiten Orthesengelenks 6 und somit zur Anpassung der zweiten Drehachse 7 aufweist. Stattdessen ist bei der Orthese 100 der Verbindungspunkt 12 zwischen dem Ringbügel 8 und der Längsschiene 11 festgelegt.

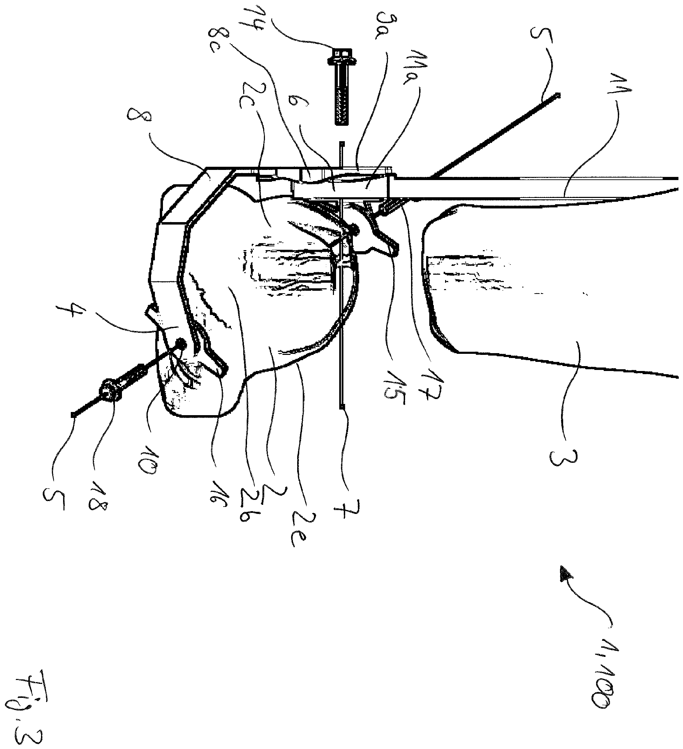

Figur 3 zeigt die Orthesen 1 und 100 aus Figur 1 und Figur 2 in einer Hinteransicht mit Blick auf den Fersenbereich 2b des Fußteils 2. In Figur 3 ist der Verlauf der zweiten Drehachse 7 zu erkennen. Die zweite Drehachse 7 verläuft in etwa senkrecht zu den Längsachsen des Fußteils 2 und des Unterschenkelteils 3. Die zweite Drehachse verläuft somit auch senkrecht zur Sagittalebene durch den Fußteil 2, welche den Fußteil 2 längs in zwei Hälften teilt. Die erste Drehachse 5 verläuft wie auch in den Figuren 1 und 2 erkennbar von oberhalb eines Großzehenbereichs 2d auf der Fußteilinnenseite 2c zum unteren, äußeren Fersenbereich 2b.

Ferner ist in Figur 3 zu erkennen, dass das zweite Orthesengelenk 6 an der Ausbuchtung 8a des Ringbügels 8 auf eine dem Fersenbereich 2b zugewandten Seite ein hinteres Anschlagelement 8c angeordnet ist, welches eine Drehbewegung der Längsschiene 11 um die zweite Drehachse 7 in Richtung des Fersenbereichs 2b beschränkt.

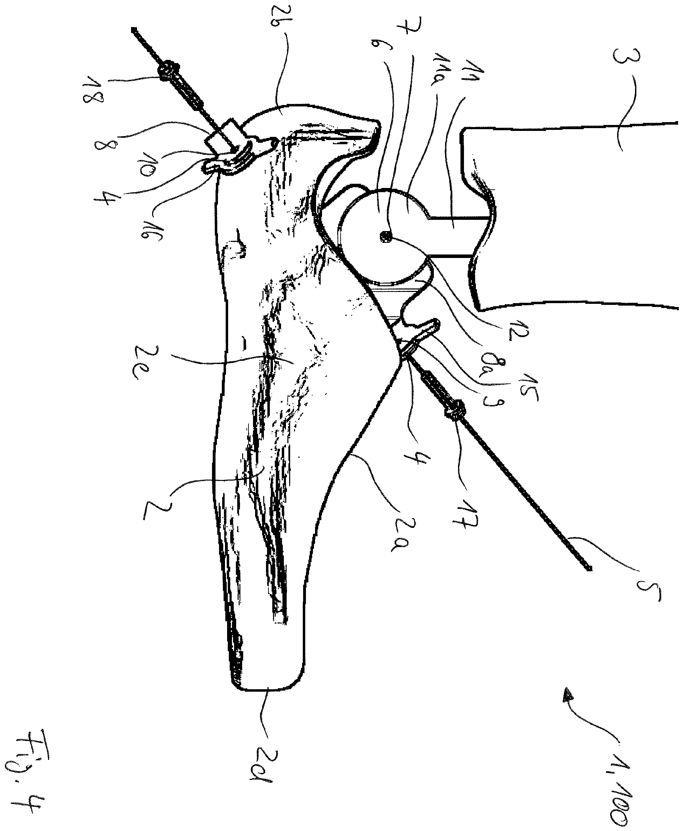

Figur 4 zeigt die Orthesen 1 und 100 aus Figuren 1 und 2 in einer Seitenansicht mit Blick auf eine Außenseite 2e des Fußteils 2. Ebenso wie eine Anordnung des Ringbügels 8, der Längsschiene 11 und des zweiten Orthesengelenks 6 auf einer der Köpermitte zugewandten Innenseite der Orthese 1 oder 100 wäre es denkbar, den Ringbügel 8, die Längsschiene 11 und das zweite Orthesengelenk 6 auf einer der Körpermitte abgewandten Außenseite der Orthese anzuordnen.

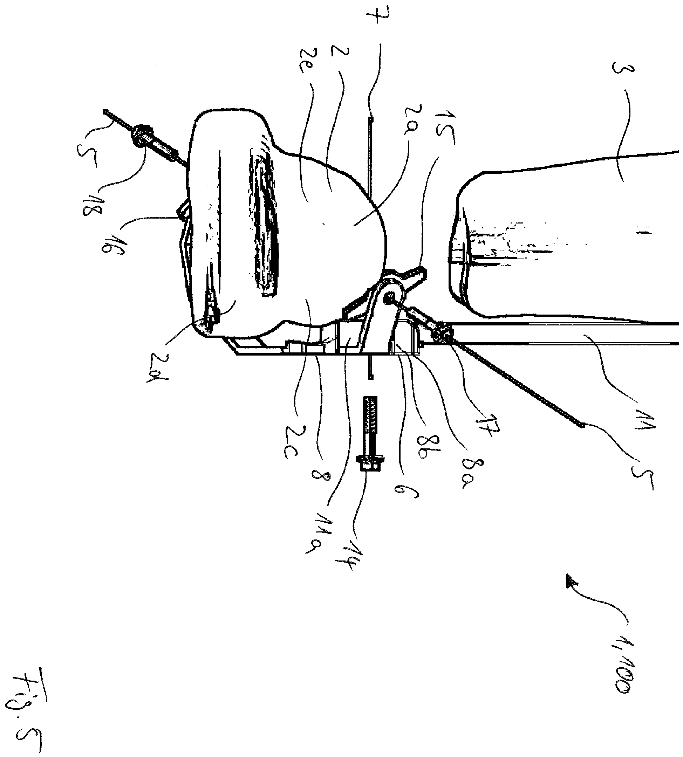

Figur 5 zeigt die Orthesen 1 und 100 aus Figuren 1 und 2 in einer Vorderansicht mit Blick auf einen Zehenbereich des Fußteils 2. In Figur 5 ist zu erkennen, dass an einer dem Großzehenbereich 2d zugewandten Seite der Ausbuchtung 8a des Ringbügels 8 ein vorderes Anschlagelement 8b des zweiten Orthesengelenks 6 angeordnet ist. Dieses vordere Anschlagelement 6 beschränkt eine Drehbewegung der Längsachse 11 um die zweite Drehachse 7 in Richtung des Großzehenbereichs 2d.

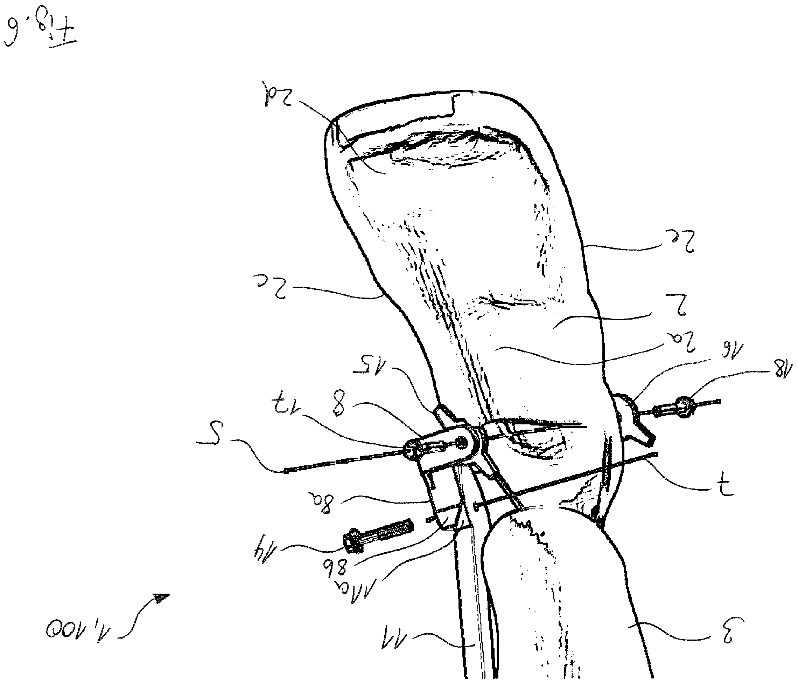

Figur 6 zeigt eine Draufsicht der Orthesen 1 und 100 aus Figuren 1 und 2 mit Blick auf den Fußteil 2. Wie in Figur 6 zu erkennen ist, ist das vordere Anschlagelement 8b als Teil der Ausbuchtung 8a des Ringbügels 8 ausgeformt, welches etwa im rechten Winkel zur Ausbuchtung 8a in Richtung des Fußteils 2 umgeformt ist, sodass es um den Rand des kreisscheibenförmigen Endes 11a der Längsachse 11 greift.

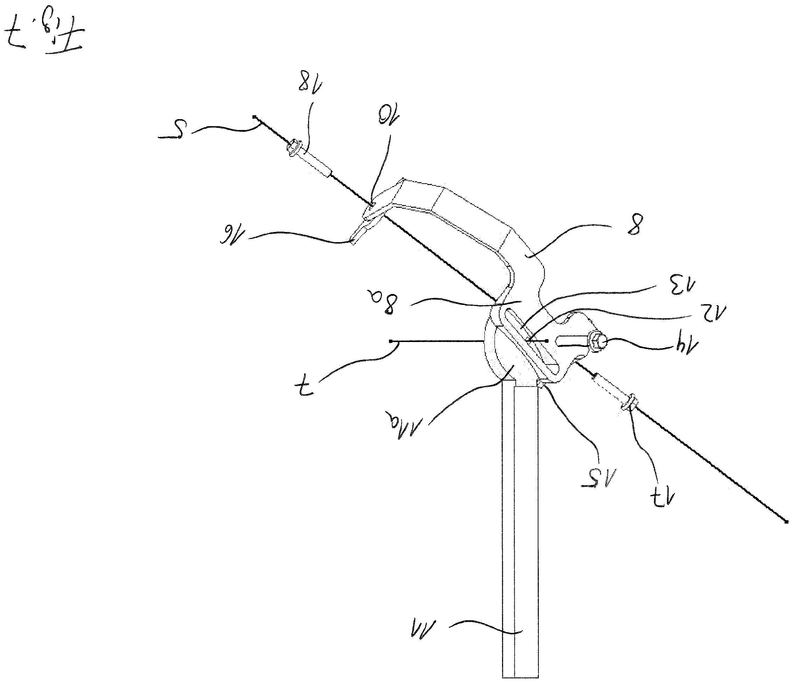

Figur 7 zeigt eine Anordnung des ersten Orthesengelenks 4 mit Ringbügel 8 und Abschlagelementen 15 und 16 und des zweiten Orthesengelenks 6 mit Ausbuchtung 8a und Längsschiene 11 gemäß dem ersten Ausführungsbeispiel in einer perspektivischen Detailansicht. Wie bereits in Figur 1 dargestellt weist die Ausbuchtung 8a des Ringbügels 8 die Nut 13 zur Verschiebung und Anpassung der Position der zweiten Drehachse 7 auf. Die gewählte Position der zweiten Drehachse 7 kann dann mittels der Schraube 14 fixiert werden.

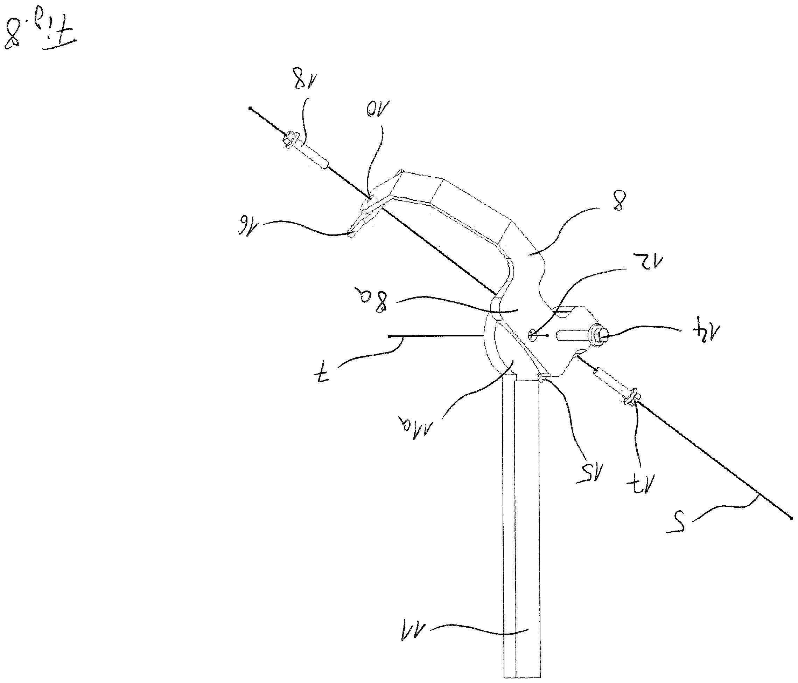

Figur 8 zeigt eine Anordnung des ersten Orthesengelenks 4 mit Ringbügel 8 und Abschlagelementen 15 und 16 und des zweiten Orthesengelenks 6 mit Ausbuchtung 8a und Längsschiene 11 gemäß dem zweiten Ausführungsbeispiel in einer perspektivischen Detailansicht. Im Gegensatz zum in Figuren 1 und 7 dargestellten ersten Ausführungsbeispiel weist die Ausbuchtung 8a des Ringbügels 8 im zweiten Ausführungsbeispiel keine Nut 13 zur Verschiebung und Anpassung der zweiten Drehachse 7 auf. Bei der Ausbuchtung 8a gemäß dem zweiten Ausführungsbeispiel ist die Position der Drehachse 7 durch den Verbindungspunkt 12 festgelegt. Die Schraube 14 dient hier lediglich zur Sicherung des Orthesengelenks 6.

## Patentansprüche

1. Orthese (1) zur Beübung eines ein oberes und ein unteres Sprunggelenk aufweisenden menschlichen Sprunggelenks in einer korrigierten Stellung, umfassend
einen Fußteil (2) zur Aufnahme eines Fußes, wobei der Fußteil (2) eine Fußteiloberseite (2a) und einen unteren Fersenbereich (2b) aufweist,
einen Unterschenkelteil (3) zur Aufnahme eines Unterschenkels, wobei
der Fußteil (2) und der Unterschenkelteil (3) derart zueinander ausgerichtet sind, dass das Sprunggelenk in eine korrigierte Stellung bringbar ist, und
mindestens ein erstes Orthesengelenk (4),
wobei das erste Orthesengelenk (4) eine Drehung des Fußteils (2) relativ zum Unterschenkelteil (3) um eine erste Drehachse (5) ermöglicht, wobei die erste Drehachse (5) den Fußteil (2) an der Fußteiloberseite (2a) und in dem unteren Fersenbereich (2b) schneidet,
und wobei die erste Drehachse (5) einer Drehachse des unteren Sprunggelenks in der korrigierten Stellung des Sprunggelenks entspricht,
***dadurch gekennzeichnet, dass***
das erste Orthesengelenk (4) einen Ringbügel (8) umfasst, an dessen Enden der Fußteil (2) um die erste Drehachse (5) drehbar befestigt ist.

2. Orthese (1) nach dem vorhergehenden Anspruch, **dadurch gekennzeichnet, dass** die erste Drehachse (5) in einem Winkel zwischen 30° und 50°, vorzugsweise zwischen 39° und 42°, in Richtung einer Fußteiloberseite (2a) zu einer Horizontalebene durch den Fußteil (2) und in einem Winkel zwischen 10° und 30°, vorzugsweise zwischen 12° und 16°, in Richtung einer Fußteilinnenseite (2c) zu einer Sagittalebene durch den Fußteil (2) durch einen Fersenbereich des Fußteils (2) verläuft, in dem das untere Sprunggelenk positionierbar ist.

3. Orthese (1) nach einem der vorhergehenden Ansprüche, **dadurch gekennzeichnet, dass** der Ringbügel (8) starr mit dem Unterschenkelteil (3) verbunden ist.

4. Orthese (1) nach einem der vorhergehenden Ansprüche, **gekennzeichnet durch** ein zweites Orthesengelenk (6), welches eine Drehung des Fußteils (2) relativ zum Unterschenkelteil (3) um eine zweite Drehachse (7) ermöglicht, wobei die zweite Drehachse (7) einer Drehachse des oberen Sprunggelenks in der korrigierten Stellung des Sprunggelenks entspricht.

5. Orthese (1) nach dem vorhergehenden Anspruch, **dadurch gekennzeichnet, dass** die zweite Drehachse (7) weitgehend senkrecht, vorzugsweise in einem Winkel zwischen 70° und 90°, insbesondere in einem Winkel von 80° zu einer Längsachse des Unterschenkelteils (3) durch einen Knöchelbereich der Orthese (1) verläuft, in dem das obere Sprunggelenk positionierbar ist.

6. Orthese (1) nach einem der zwei vorhergehenden Ansprüche, **dadurch gekennzeichnet, dass** der Ringbügel (8) über das zweite Orthesengelenk (6) mit dem Unterschenkelteil (3) verbunden ist.

7. Orthese (1) nach dem vorhergehenden Anspruch, **dadurch gekennzeichnet, dass** der Unterschenkelteil (3) über eine Längsschiene (11) mit dem Ringbügel (8) verbunden ist, wobei das zweite Orthesengelenk (6) in einem Verbindungspunkt (12) zwischen der Längsschiene (11) und dem Ringbügel (8) angeordnet ist.

8. Orthese (1) nach einem der vorhergehenden Ansprüche, **dadurch gekennzeichnet, dass** das erste (4) und/oder das zweite Orthesengelenk (6) Anschlagelemente (8b, 8c) zur Einschränkung einer Bewegung des Fuß- (2) und des Unterschenkelteils (3) zueinander um die erste (5) und/oder die zweite Drehachse (7) aufweist.

9. Orthese (1) nach einem der vorhergehenden Ansprüche, **dadurch gekennzeichnet, dass** das erste Orthesengelenk (4) und/oder das zweite Orthesengelenk (6) ein Kugelgelenk oder ein Scharniergelenk aufweist.

10. Orthese (1) nach einem der vorhergehenden Ansprüche, **dadurch gekennzeichnet, dass** das erste Orthesengelenk (4) und/oder das zweite Orthesengelenk (6) mit einem Motor zu einer elektronisch gesteuerten Bewegung des Fuß- (2) und Unterschenkelteils (3) zueinander um die erste (5) und/oder die zweite Drehachse (7) gekoppelt ist.

## Claims

1. An orthosis (1) for exercising a human ankle joint having an upper and a lower ankle joint in a corrected position comprising
a foot part (2) for receiving a foot, wherein the foot part (2) has a foot part upper side (2a) and a lower heel region (2b);
a lower leg part (3) for receiving a lower leg, wherein
the foot part (2) and the lower leg part (3) are aligned with respect to one another such that the ankle joint can be moved into a corrected position; and
at least one first orthosis joint (4);
wherein the first orthosis joint (4) enables a rotation of the foot part (2) relative to the lower leg part (3) about a first axis of rotation (5), with the first axis of rotation (5) intersecting the foot part (2) at the foot part upper side (2a) and in the lower heel region (2b);
and wherein the first axis of rotation (5) corresponds to an axis of rotation of the lower ankle joint in the corrected position of the ankle joint,
***characterized in that***
the first orthosis joint (4) comprises a ring strap (8) to whose ends the foot part (2) is fastened rotatably about the first axis of rotation (5).

2. An orthosis (1) in accordance with the preceding claim, **characterized in that** the first axis of rotation (5) extends at an angle between 30° and 50°, preferably between 39° and 42°, in the direction of a foot part (2a) upper side with respect to a horizontal plane through the foot part (2) and at an angle between 10° and 30°, preferably between 12° and 16°, in the direction of an inner foot part side (2c) with respect to a sagittal plane through the foot part (2) through a heel region of the foot part (2) in which the lower ankle joint is positionable.

3. An orthosis (1) in accordance with one of the preceding claims, **characterized in that** the ring strap (8) is rigidly connected to the lower leg part (3).

4. An orthosis (1) in accordance with one of the preceding claims, **characterized by** a second orthosis joint (6) that enables a rotation of the foot part (2) relative to the lower leg part (3) about a second axis of rotation (7), with the second axis of rotation (7) corresponding to an axis of rotation of the upper ankle joint in the corrected position of the ankle joint.

5. An orthosis (1) in accordance with the preceding claim, **characterized in that** the second axis of rotation (7) extends in a substantially perpendicular manner, preferably at an angle between 70° and 90°, in particular at an angle of 80°, relative to a longitudinal axis of the lower leg part (3) through an ankle region of the orthosis (1) in which the upper ankle joint is positionable.

6. An orthosis (1) in accordance with one of the two preceding claims, **characterized in that** the ring strap (8) is connected to the lower leg part (3) via the second orthosis joint (6).

7. An orthosis (1) in accordance with the preceding claim, **characterized in that** the lower leg part (3) is connected to the ring strap (8) via a longitudinal splint (11), with the second orthosis joint (6) being arranged at a point of connection (12) between the longitudinal splint (11) and the ring strap (8).

8. An orthosis (1) in accordance with one of the preceding claims, **characterized in that** the first (4) and/or second orthosis joint (6) has/have abutment elements (8b, 8c) to restrict a movement of the foot part (2) and of the lower leg part (3) with respect to one another about the first (5) and/or second axis of rotation (7).

9. An orthosis (1) in accordance with one of the preceding claims, **characterized in that** the first (4) and/or second orthosis joint (6) has/have a spherical joint or a hinge joint.

10. An orthosis (1) in accordance with one of the preceding claims, **characterized in that** the first orthosis joint (4) and/or the second orthosis joint (6) is/are coupled to a motor for an electronically controlled movement of the foot part (2) and of the lower leg part (3) with respect to one another about the first (5) and/or second axis of rotation (7).

## Revendications

1. Orthèse (1) pour l'entraînement d'une cheville humaine comprenant une cheville supérieure et une cheville inférieure, dans une position corrigée, comprenant
une partie de pied (2) pour le logement d'un pied, la partie de pied (2) comprenant un côté supérieur de la partie de pied (2a) et une partie de talon inférieure (2b),
une partie de jambe inférieure (3) pour le logement d'une jambe,
la partie de pied (2) et la partie de jambe (3) étant orientées l'une par rapport à l'autre de façon à ce que la cheville puisse être amenée dans une position corrigée et
au moins une première articulation d'orthèse (4),
la première articulation d'orthèse (4) permettant une rotation de la partie de pied (2) par rapport à la partie de jambe (3) autour d'un premier axe de rotation (5), le premier axe de rotation (5) coupant la partie de pied (2) au niveau du côté supérieur de la partie de pied (2a) et dans la partie de talon inférieure (2b),
et le premier axe de rotation (5) correspondant à un axe de rotation de la cheville inférieure dans la position corrigée de la cheville,
**caractérisée en ce que**
la première articulation d'orthèse (4) comprenant un étrier annulaire (8), au niveau de l'extrémité duquel la partie de pied (2) est fixée de manière rotative autour du premier axe de rotation (5).

2. Orthèse (1) selon la revendication précédente, **caractérisée en ce que** le premier axe de rotation (5) s'étend avec un angle entre 30° et 50°, de préférence entre 39° et 42°, en direction d'un côté supérieur de partie de pied (2a) par rapport à un plan horizontal traversant la partie de pied (2) et s'étend avec un angle entre 10° et 30°, de préférence entre 12° et 16°, en direction d'un côté intérieur de la partie de pied (2c) par rapport à un plan sagittal traversant la partie de pied (2) à travers une partie de talon de la partie de pied (2), dans lequel la cheville inférieure peut être positionnée.

3. Orthèse (1) selon l'une des revendications précédentes, **caractérisée en ce que** l'étrier annulaire (8) est relié de manière rigide avec la partie de jambe (3).

4. Orthèse (1) selon l'une des revendications précédentes, **caractérisée par** une deuxième articulation d'orthèse (6), qui permet une rotation de la partie de pied (2) par rapport à la partie de jambe (3) autour d'un deuxième axe de rotation (7), le deuxième axe de rotation (7) correspondant à un axe de rotation de la cheville supérieure dans la position corrigée de la cheville.

5. Orthèse (1) selon la revendication précédente, **caractérisée en ce que** le deuxième axe de rotation (7) s'étend de manière largement perpendiculaire, de préférence avec un angle entre 70° et 90°, plus particulièrement avec un angle de 80° par rapport à un axe longitudinal de la partie de jambe (3) traversant une partie de cheville de l'orthèse (1), dans lequel la cheville supérieure peut être positionnée.

6. Orthèse (1) selon l'une des deux revendications précédentes, **caractérisée en ce que** l'étrier annulaire (8) est relié par l'intermédiaire de la deuxième articulation d'orthèse (6) avec la partie de jambe (3).

7. Orthèse (1) selon la revendication précédente, **caractérisée en ce que** la partie de jambe (3) est reliée par l'intermédiaire d'un rail longitudinal (11) avec l'étrier annulaire (8), la deuxième articulation d'orthèse (6) étant disposée au niveau d'un point de liaison entre le rail longitudinal (11) et l'étrier annulaire (8).

8. Orthèse (1) selon l'une des revendications précédentes, **caractérisée en ce que** la première (4) et/ou la deuxième articulation d'orthèse (6) comprend des éléments de butée (8b, 8c) pour la limitation d'un mouvement de la partie de pied (2) et de la partie de jambe (3) entre elles autour du premier (5) et/ou du deuxième axe de rotation (7).

9. Orthèse (1) selon l'une des revendications précédentes, **caractérisée en ce que** la première (4) et/ou la deuxième articulation d'orthèse (6) comprend une articulation sphérique ou une articulation à charnière.

10. Orthèse (1) selon l'une des revendications précédentes, **caractérisée en ce que** la première (4) et/ou la deuxième articulation d'orthèse (6) est couplée avec un moteur pour un mouvement contrôlé électriquement de la partie de pied (2) et de la partie de jambe (3) entre elles autour du premier (5) et/ou deuxième axe de rotation (7).
